# EUROPÄISCHE PATENTSCHRIFT

(11) **EP 1 105 058 B1**
(45) Veröffentlichungstag und Bekanntmachung des Hinweises auf die Patenterteilung: **26.06.2002**
(21) Anmeldenummer: 98938569.5
(22) Anmeldetag: 21.08.1998
(51) Int. Cl.: A61B 17/70, A61B 17/86, A61F 2/44

(54) **SELBSTSCHNEIDENDES HOHLZYLINDRISCHES KNOCHENVERANKERUNGSELEMENT**
SELF-CUTTING HOLLOW CYLINDRICAL BONE ANCHORING ELEMENT
ELEMENT D'ANCRAGE OSSEUX SOUS FORME DE CYLINDRE CREUX AUTOCOUPANT

(43) Veröffentlichungstag der Anmeldung: 13.06.2001
(73) Patentinhaber: SYNTHES AG Chur, 7002 Chur (CH)
(72) Erfinder: STEINER, Beatrice, CH-6330 Cham (CH); AEBI, Max, Outremont, Québec H2V 4P1 (CA)
(74) Vertreter: Lusuardi, Werther Giovanni, Dr.
(86) Internationale Anmeldenummer: CH9800359
(87) Internationale Veröffentlichungsnummer: WO0010473

(56) Entgegenhaltungen:
- DE-U- 29 710 979
- GB-A- 231 155
- GB-A- 2 294 399
- US-A- 4 537 185
- US-A- 5 129 901

## Beschreibung

Die Erfindung bezieht sich auf ein Knochenverankerungselement gemäss dem Oberbegriff des Patentanspruchs 1 und auf eine Vorrichtung zur Knochenfixation gemäss dem Oberbegriff des Patentanspruchs 9.

Zur Fixation von Knochenteilen oder speziell auch bei Wirbelsäulenfixationen werden die Knochenteile oder Wirbelkörper mittels Knochenschrauben, Pedikelschrauben oder anderen Knochenverankerungselementen an internen Platten oder Stäben fixiert.

Ein Implantat zur relativen Fixierung von Knochenteilen oder auch zur Wirbelsäulenfixation ist in der DE 297 10 979 AESCULAP offenbart. Das Implantat umfasst in jedes Knochenteil einsetzbare Verankerungselemente, Verbindungselemente, woran die Verankerungselemente mittels einer lösbaren Kugelklemmverbindung befestigbar sind, und Längsträger, die ebenfalls an den Verbindungselementen festgeklemmt werden und mittels welcher mehrere Verankerungselemente, die in verschiedenen Knochenteile oder auch Wirbelkörper eingesetzt sind, starr verbindbar sind. Die Verankerungselemente sind als hohlzylindrische Knochenschrauben ausgebildet und weisen auf dem Aussenmantel ein Gewinde sowie zwischen den Gewindegängen liegende radiale Durchgangsöffnungen auf. Vor dem Einsetzen dieser hohlzylindrischen Verankerungselemente muss in den Knochen ein Aufnahmekanal gebohrt oder gefräst werden.

Ein anderes Implantat, welches eine hohlzylindrische Knochenschraube umfasst, ist in der US 5,015,247 MICHELSON offenbart. Wie das oben erwähnte Verankerungselement besteht diese Knochenschraube, welche vor allem zum Einsetzen in den Zwischenwirbelraum gestaltet ist, aus einem Hohlzylinder mit einem Aussengewinde und zwischen den Gewindegängen angeordneten radialen Durchgangsöffnungen. Dieses Implantat wird entweder in einen ebenfalls ausgebohrten oder ausgefräste Knochenkanal oder in eine Bohrung im Knochen eingeschraubt. Im letzteren Fall wird der Hohlraum in der Knochenschraube mit körpereigenen Knochenspänen aufgefüllt, so dass die Fusion zwischen den benachbarten Wirbelkörpern selbst und zwischen Wirbelkörpern und Implantat gefördert wird.

Beide erwähnte Implantate weisen den Nachteil auf, dass vorgängig zum Einschrauben der hohlzylindrischen Knochenschraube oder des Verankerungselementes ein Kanal oder eine Bohrung in den Knochen gefräst oder gebohrt werden muss.

Hier will die Erfindung Abhilfe schaffen. Der Erfindung liegt die Aufgabe zugrunde, ein selbstschneidendes Verankerungselement zu schaffen. Durch diese selbstschneidende Eigenschaft des Knochenverankerungselementes ergibt sich eine wesentlich kürzere Implantationsdauer.

Eine durchbohrte Fixationsschraube mit einem kreiszylindrischen Verankerungsteil, einem Verbindungselement am oberen Ende und einem selbstschneidenden Aussengewinde auf der Mantelfläche des Verankerungsteils ist aus der US 4,537,185 STEDNITZ bekannt. Diese bekannte Fixationsschraube ist axial durchbohrt und mit Schneidezähnen am vorderen Ende versehen.

Eine weitere durchbohrte, orthopädische Fixationsschraube mit einem kreiszylindrischen Verankerungsteil und einem selbstschneidenden Aussengewinde auf der Mantelfläche des Verankerungsteiles ist aus der US 5,129,901 DECOSTE bekannt. Auch diese bekannte Fixationsschraube ist axial durchbohrt und mit Schneidezähnen am vorderen Ende versehen.

Bei beiden oben erwähnten Fixationsschrauben reichen die Aussengewinde bis oder knapp bis an die Schraubenspitzen und werden somit in oder auch in den spongiösen Bereich des Knochens geschraubt. Nachteilig daran ist, dass bei osteoporotischen Erkrankungen, wo die Kortikalis fast normal bestehen bleibt und sich die Spongiosa aber zurückbildet, eine Verankerung mittels eines Spongiosagewindes nicht ausführbar ist. Zudem wird bei beiden oben erwähnten Fixationsschrauben die selbstschneidende Eigenschaft der Aussengewinde mit axialen Rillen hergestellt, wodurch die Fixationsschrauben vorne keine Mantelflächen mit einer glatten Oberfläche aufweisen.

Die Erfindung löst die gestellte Aufgabe mit einem Knochenverankerungselement, welches die Merkmale des Anspruchs 1 aufweist, sowie mit einer Vorrichtung zur Knochenfixation, welche die Merkmale des Anspruchs 9 aufweist.

Weitere vorteilhafte Ausgestaltungen der Erfindung sind in den abhängigen Ansprüchen gekennzeichnet.

In einer bevorzugten Ausführungsform des erfindungsgemässen Knochenverankerungselementes besteht dieses aus einem kreiszylindrischen Verankerungsteil, welches am einen Ende stirnseitig tangential angeordnete Schneidezähne aufweist. Das Verankerungselement ist hohlzylindrisch ausgebildet und auf der äusseren Mantelfläche mit einem selbstformenden Gewinde versehen. Mittels der Schneide- oder Sägezähne lässt sich das Verankerungsteil ohne vorgängige Bohrung oder Ausfräsung eines Knochenkanals direkt in den Knochen einbringen. Die Knochenspäne werden in die Bohrung des Hohlzylinders abgeführt.

Die Anzahl der Schneidezähne beträgt zwischen 10 bis 40, vorzugsweise zwischen 20 bis 30 Zähnen.

Zudem weisen die Schneidezähne einen Spanwinkel von 10° bis 40°, vorzugsweise von 25° bis 35° auf. Wobei als Spanwinkel derjenige Winkel verstanden wird, der zwischen der Längsachse des hohlzylindrischen Verankerungselementes und der Schnittfläche eines Schneidezahnes liegt. Der Freiwinkel beträgt sinnvollerweise zwischen 5° und 40°, vorzugsweise zwischen 18° und 28°.

Zudem schliesst die Schnittkante mit dem Lot zur Längsachse einen Winkel zwischen 30° und 60°, vorzugsweise zwischen 40° und 50° ein, wobei sich die Schneidenecke am äusseren Umfang des Verankerungselementes befindet.

An anderen Ende ist das Verankerungselement mit einem Verbindungselement zur Kupplung an ein anderes implantierbares Element, beispielsweise an eine Verbindungsplatte, eine Verstellplatte oder an Längsträgern, versehen. In der einfachsten Ausführungsform kann dieses Verbindungselement aus einem Zylinder, welcher in eine entsprechende Bohrung in der Platte einsteckbar ist, bestehen. Vorteilhafterweise ist das Verbindungselement jedoch so ausgebildet, dass das Verankerungselement in einem Winkelbereich schwenkbar in der Platte fixierbar ist. Diese Schwenkbarkeit lässt sich beispielsweise durch eine kugelförmige Ausführung des Verbindungselementes mit entsprechenden Ausnehmungen in der Platte herstellen.

Die Mantelfläche des Verankerungselementes weist gemäß der Erfindung vom unteren, mit den Schneidezähnen versehenen Ende her auf einer Teillänge zwischen 50% und 85% der Höhe H eine glatte Oberfläche auf. Vorteile bietet die Erfindung dadurch, dass die Seitenwand des hohlzylindrischen Verankerungsteiles auf dieser Teillänge mit radialen Durchgangsbohrungen ausgestattet werden kann, welche eine Fusion des aussen am Verankerungsteil liegenden Knochens mit dem innerhalb des Verankerungsteils liegenden Kerns des Knochens ermöglichen. Zudem wird durch die Durchgangsbohrungen Material eingespart, wodurch das Gewicht des Implantates erheblich abgesenkt werden kann. Diese Teillänge L beträgt sinnvollerweise zwischen 60% und 85%, vorzugsweise zwischen 70% und 80% der Höhe H der Verankerungsteils.

In einer bevorzugten Ausführungsform der erfindungsgemässen Vorrichtung umfasst diese mindestens zwei Knochenverankerungselemente, mindestens eine Platte mit einer Zentralachse, wobei diese mindestens eine Platte mit Mitteln zur Aufnahme der Verbindungselemente der beiden Knochenverankerungselemente versehen ist. Die Mittel zur Aufnahme dieser Verbindungsmittel bestehen im wesentlichen aus Bohrungen mit geeigneten Längsguerschnitten in der Platte, welche gestatten, dass die an den Verankerungselementen angebrachten Verbindungsmittel darin gelagert werden und mit Mitteln zur Fixierung der Knochenverankerungselemente in der oder den Platten lösbar befestigt werden. Die Mittel zur Fixierung der Knochenverankerungselemente bestehen in bevorzugten Ausführungsformen der erfindungsgemässen Vorrichtung aus Schrauben oder Muttern, die in oder über die Verbindungselemente schraubbar sind. Zudem sind die Mittel zur Aufnahme der Verbindungsmittel, beispielsweise in Form von entlang der Zentralachse der Platte verlaufenden Längslöchern so gestaltet, dass die beiden Knochenverankerungselemente in Richtung der Zentralachse um einen Abstand Z, welcher 10 und 80 mm, vorzugsweise zwischen 20 und 60 mm beträgt, gegeneinander verschiebbar sind.

Eine weitere Ausführungsform der erfindungsgemässen Vorrichtung zeichnet sich dadurch aus, dass die Vorrichtung zwei in Richtung der Zentralachse verschiebbare Platten, wobei jede der beiden Platten mit Mitteln zur Aufnahme eines Verbindungselementes versehen ist, und ein Feststellmittel zur relativen Fixierung der beiden Platten umfasst. Dieses Feststellmittel ist in den verschiedenen Varianten als Schraube, welche die eine der Platten durch eine Bohrung durchdringt und in die andere Platte eingeschraubt ist, ausgeführt.

Durch die geeignete Gestaltung der Verbindungselemente, z.B. in der Ausführung als kugelförmige Verbindungselemente wird ermöglicht, dass zwischen der Zentralachse der mindestens einen Platte und jeder der Längsachsen der Verankerungsteile ein Winkel zwischen 60° und 120°, vorzugsweise zwischen 70° und 110° einstellbar ist.

Ebenfalls ist eine Ausführung der erfindungsgemässen Vorrichtung vorgesehen, bei der die beiden Platten an den mit einander in Kontakt stehenden Flächen mit einer Verzahnung versehen sind. Dadurch wird die Vorrichtung gegen Verrutschen der beiden Platten gegeneinander gesichert und stabiler. Zudem werden die beiden Platten durch Nasen oder Führungsbacken auf der Seite gegen Verdrehen gesichert.

Eine weitere Ausführungsform der erfindungsgemässen Vorrichtung besteht darin, dass anstelle einer zweiten Platte zwei kreisringförmige Scheiben vorgesehen sind, deren eine unterhalb der Platte zu liegen kommt und zur Aufnahme der Verbindungsmittel des Verankerungselementes dient und deren andere zusammen mit einer in diese einbringbaren Schraube zur Herstellung einer klemmbaren Fixierung des ersten Verankerungselementes gegenüber der Platte dient. Das zweite Verankerungselement wird in der Platte fixiert. Zur Herstellung des variablen Abstandes zwischen den Verankerungselementen ist mindestens eine der Bohrungen zur Aufnahme der Verbindungselemente als Langloch ausgebildet. Die unterhalb der Platte liegende Scheibe und die Platte selbst können auf den Berührungsflächen ebenfalls mit einer Rasterung versehen sein.

Anstelle des kugelförmigen Verbindungselementes kann durch eine auf der Seite des Verankerungselementes auf der Platte liegende, einseitig konvex ausgebildete Scheibe und eine dazu korrespondierende konkave Ausbildung des oberen Endes des Verankerungselementes die Schwenkbarkeit des Verankerungselementes ergeben.

Die durch die Erfindung erreichten Vorteile sind im wesentlichen darin zu sehen, dass dank des erfindungsgemässen Knochenverankerungselementes am Knochen vor dem Einbringen des Implantates keine Bohrungen oder Ausfräsungen vorgenommen werden müssen.

Die Erfindung und Weiterbildungen der Erfindung werden im folgenden anhand der teilweise schematischen Darstellungen eines Ausführungsbeispieles noch näher erläutert.
Es zeigen:
Fig. 1 eine schematische, perspektivische Darstellung einer Ausführungsform des erfindungsgemässen Knochenverankerungselementes;
Fig. 2 einen Ausschnitt eines Schneidezahnes gemäss einer Ausführungsform der erfindungsgemässen Vorrichtung;
Fig. 3 einen Aufriss einer Ausführungsform der erfindungsgemässen Vorrichtung; und
Fig. 4 eine Aufsicht auf die in Fig. 3 dargestellte Ausführungsform der erfindungsgemässen Vorrichtung.

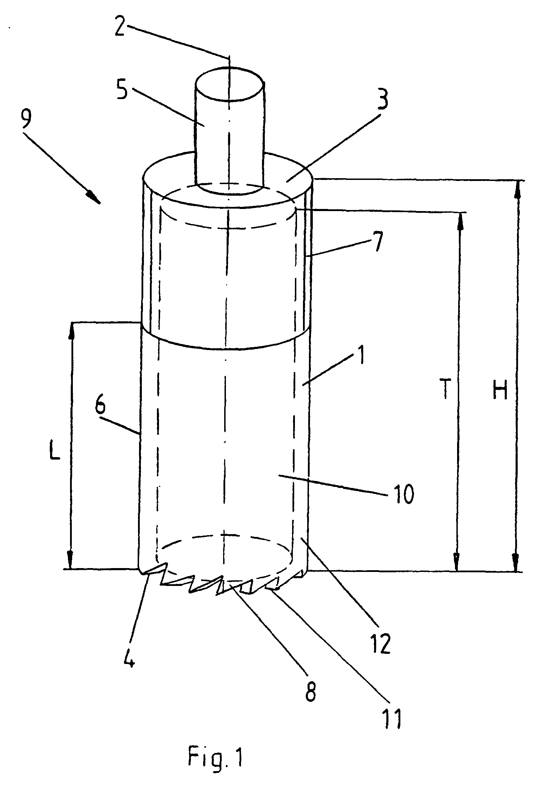

In Fig. 1 ist schematisch eine Ausführungsform des erfindungsgemässen Knochenverankerungselementes 9 dargestellt. Dieses umfasst einen kreiszylinderisches Verankerungselement 1 mit der Höhe H und koaxial zu dessen Längsachse 2 ein konzentrisch angeordnetes Verbindungselement 5, welches in dieser Ausführungsform als einfacher zylindrischer Zapfen dargestellt ist. Anstelle des zylindrischen Zapfens könnte das Verbindungselement 5 auch kugelförmig ausgebildet sein. Das Verbindungselement 5 schliesst in Richtung der Längsachse 2 direkt an das obere Ende 3 des Verankerungselementes 1 an. Vom unteren Ende 4 des Verankerungselementes 1 her dringt konzentrisch eine Bohrung 10 mit einer Tiefe T, welche geringer als die Höhe H ist, in das Verankerungselement 1 ein. Somit ergibt sich auf einer Länge, die der Tiefe T entspricht, ein hohlzylindrischer Querschnitt des Verankerungselementes 1. Am unteren Ende 4 sind auf dem Hohlzylinder stirnseitig Schneideoder Sägezähne 8 tangential angeordnet. Die Schnittkanten 11 der in dieser Ausführungsform vorgesehenen Schneidezähne 8 verlaufen radial. Auf einer am unteren Ende 4 beginnenden Teillänge L, welche ebenfalls geringer als die Höhe H ist, ist die äussere Mantelfläche 6 der Verankerungselementes 1 glatt, währenddem der übrig bleibenden Teil der Höhe H mit einem Aussengewinde 7 versehen ist. Das Knochenverankerungselement 9 kann an der Stirnfläche des Verbindungsteils 5 mit einem Innensechskant versehen sein, wodurch das Einbringen des Knochenverankerungselementes 9 in den Knochen mit einem entsprechenden Werkzeug vereinfacht wird. Anstelle des Innensechskants ist auch ein Aussensechskant am Verbindungsteil 5 oder an dem an das obere Ende 3 anschliessenden Teil des Verankerungselementes 1 möglich.

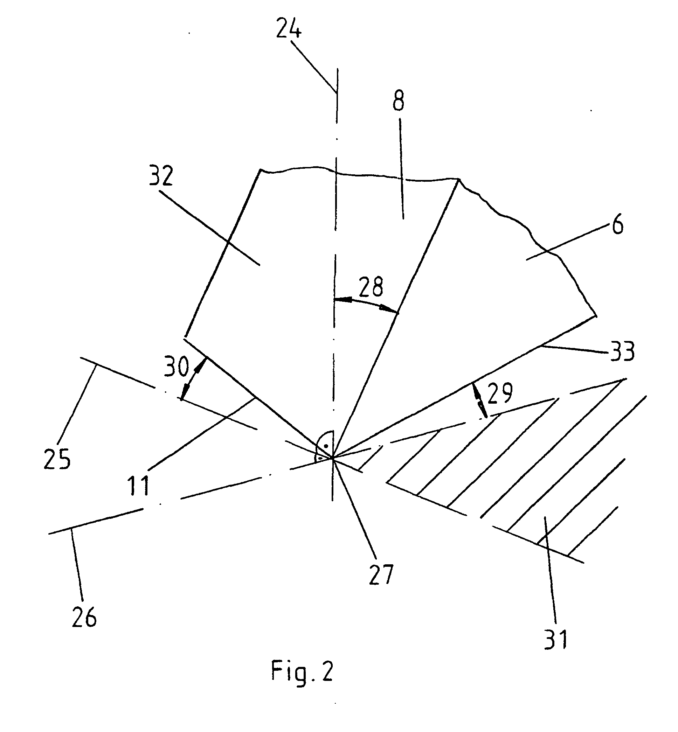

Fig. 2 zeigt einen Ausschnitt aus einem Schneidezahn 8. Eingezeichnet ist ein dreidimensionales Koordinatensystem, welches eine zur Längsachse 2 parallel z-Achse 24, eine radiale x-Achse 25 und eine zu diesem Radius rechtwinklige, zur äusseren Mantelfläche 6 tangentiale y-Achse 16 aufweist. Die Schneidenecke 27 liegt auf der äusseren Mantelfläche 6 des Verankerungsteils 1. Der Spanwinkel 28, welcher durch die z-Achse 24 und diejenige Schneidezahnfläche 32, auf welcher der Span abläuft, eingeschlossen wird, beträgt 30°. Der Freiwinkel 29 zwischen der sich peripher an der äusseren Mantelfläche 6 befindenden Schneidezahnkante 33 und der durch die x-Achse 25 und die y-Achse 26 aufgespannten Ebene 31 beträgt 22,5°.

Der Winkel 30 zwischen der radial verlaufenden Schnittkante 11 und der durch die x-Achse 25 und die y-Achse 26 aufgespannten Ebene 31 beträgt 45°, so dass die Schneidenecken 27 der Schneidezähne 8 das untere Ende 4 (Fig. 1) des Verankerungsteils 1 bilden und auf dem äusseren Umfang liegen.

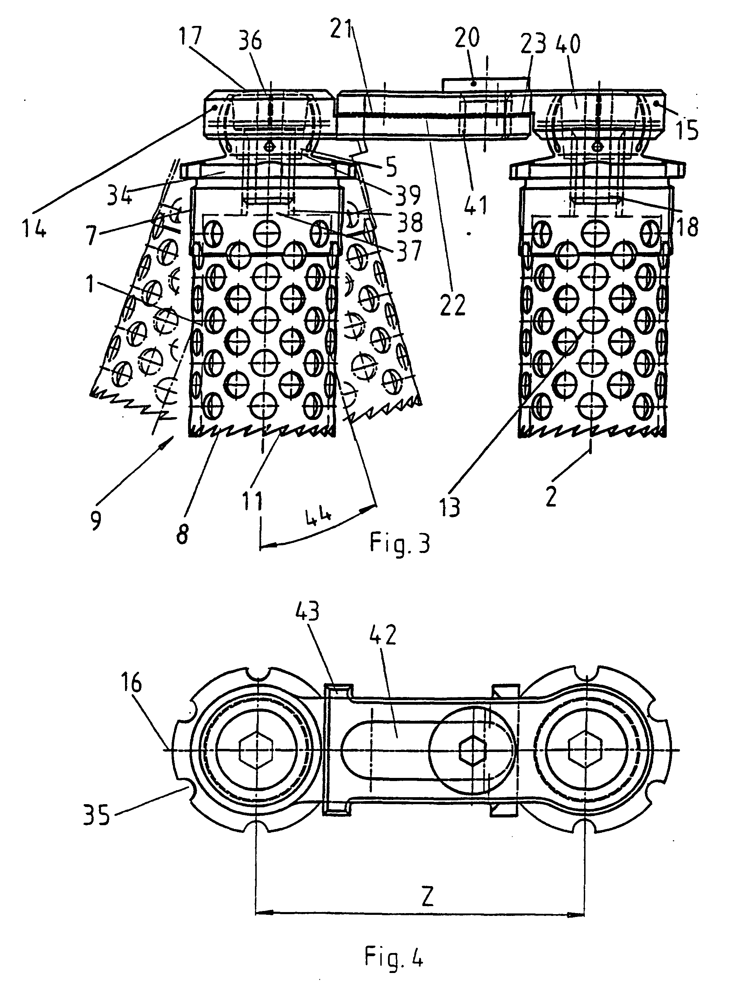

Die beiden Figuren Fig. 3 und 4 zeigen eine Ausführungsform der erfindungsgemässen Vorrichtung mit zwei Knochenverankerungselementen 9. Die beiden Knochenverankerungselemente 9 sind auf ihren hohlzylindrischen Teilen mit radialen Durchgangsbohrungen 13 versehen. Verbunden werden die beiden Knochenverankerungselemente 9 durch zwei Platten 14;15, welche eine gemeinsame Zentralachse 16 aufweisen. Die beiden Platten 14;15 überlappen sich auf in Kontakt stehenden Flächen 21;22, welche sich entlang der Zentralachse 16 erstrecken. Diese beiden miteinander in Kontakt stehenden Flächen 21;22 sind mit Verzahnungen 23 ausgerüstet, welche ein Verrutschen der beiden Platten 14;15 unter Belastung verhindert. Zudem verfügen die beiden Platten 14;15 an ihren gegeneinander gerichteten Enden über vorstehende Nasen 43, welche seitlich an den Platten 14;15 angebracht sind und sich über die Dicke der jeweils anderen Platte 14;15 erstrecken. Durch diese Nasen 43 wird verhindert, dass die Platten 14;15 sich gegeneinander verdrehen, wodurch deren Ausrichtung bezüglich der Zentralachse 16 nicht mehr gegeben wäre. Die Mittel 17 zur Aufnahme der Verbindungselemente 5 der Knochenverankerungselemente 9 sind sphärische Bohrungen. Die Verbindungselemente 5 sind als Kugelschichten gestaltet und weisen einen Durchmesser auf, der demjenigen der sphärischen Bohrungen entspricht. Die kugelschichtförmigen Verbindungselemente 5 sind mit Bohrungen 37, welche ein Innengewinde 38 und eine konische sich vom oberen Ende 3 weg verjüngende Eindrchung 39 aufweisen, ausgestattet. Die Eindrehungen 39 dienen zur Aufnahme der konischen Schraubenköpfe 40 der Schrauben, die als Mittel 18 zur Fixierung der Knochenverankerungselemente 9 an den Platten 14;15 dienen. Beim Anziehen der Schrauben werden durch die konischen Schraubenköpfe 40 die mit Schlitzen 36 und einer ebenfalls konischen Eindrehung 39 versehenen Verbindungselemente 5 gegen die Wand der als Mittel 17 zur Aufnahme der Verbindungselemente 5 dienenden sphärischen Bohrungen geklemmt. Dazu müssen die Konuswinkel des Schraubenkopfes 40 und derjenige der konischen Eindrehung 39 nicht identisch sein. Dadurch lässt sich das Knochenverankerungselement 9 in einer gegenüber dem Lot zu den Platten 14;15 um einen Winkel 44 von innen 16° und aussen 19° ausgeschwenkten Stellung in der Platte 14;15 fixieren. Das Feststellmittel 20, mittels welchem die Platten 14;15 relativ zueinander fixiert werden, ist eine Schraube, die in der unteren Platte 14 in eine Bohrung 41 mit Innengewinde eingeschraubt wird und die obere Platte 15 durch das Langloch 42 hindurch durchdringt. Damit die beiden Platten gegeneinander verschiebbar sind, wodurch der Abstand der beiden Knochenverankerungselemente 9 entlang der Zentralachse 16 variabel wird, ist als Durchdringungsöffnung für die Schraube an der oberen Platte 15 das Langloch 42 angebracht. Das Verankerungsteil 1 ist an seinem oberen Ende 3 mit einem einen grösseren Durchmesser als das Verankerungsteil 1 aufweisenden Flansch 34 versehen. Dieser Flansch 34 weist sechs in einem Winkel von 60° zueinander stehende, halbkreisförmige Einkerbungen 35 auf. Mittels dieser Einkerbungen 35 und einem dazu passenden Werkzeug kann das Knochenverankerungselement 9 in den Knochen eingedreht werden. Anstelle der Einkerbungen 35 könnte der Flansch 34 auch mit einem Aussensechskant versehen sein. Der Flansch 34 dient auch als Anschlag, so dass das Knochenverankerungselement 9 nicht zu weit in den Knochen oder Wirbelkörper eingedreht werden kann.

Zum Einsetzen des beschriebenen Implantates ist es nicht notwendig, vorher einen Aufnahmekanal in den Knochen einzuarbeiten. Vor dem Einbringen des Knochenverankerungselementes in den Knochen wird zuerst konzentrisch ein Kirschnerdraht in den Knochen eingebracht. Anschliessend wird das Knochenverankerungselement mittels eines Schlüssels, welcher im Kugelkopf des Verbindungsteils einschnappt und aussen in den sechs Einkerbungen einrastet, direkt in den Knochen oder auch Wirbelkörper eingedreht. Der Schlüssel ist durchbohrt und wird so durch den vorher eingebrachten Kirschnerdraht geführt. Der Kanal für die Aufnahme des hohlzylindrischen Verankerungsteils 1 wird durch die Schneidezähne 8 aus dem Knochen herausgearbeitet. Die dabei anfallenden Knochenspäne werden in das Innere des hohlzylindrischen Verankerungsteils 1 abgeführt. Nach dem Einschrauben des Knochenverankerungselementes 9 kann der Kirschnerdraht wieder entfernt werden. In gleicher Weise wird auch das zweite Knochenverankerungselement 9 in den Knochen eingebracht. Nach dem Einbringen der Knochenverankerungselemente 9 in die Knochenteile oder die Wirbelkörper werden die Platten 14;15 auf die Verbindungsteile 5 aufgeklickt. Durch Einschrauben und Anziehen der Schrauben mit den konischen Schraubenköpfen 40 und der Feststellschraube 20 lässt sich das gesamte Implantat in einer gewünschten Stellung blockieren.

## Patentansprüche

1. Knochenverankerungselement (9) mit
A) einem longitudinalen kreiszylindrischen Verankerungsteil (1) mit einer Längsachse (2), einem oberen Ende (3) und einem unteren Ende (4); sowie
B) einem am oberen Ende (3) angebrachten Verbindungselement (5) zur Kupplung an interne Platten oder Längsträger; wobei
C) auf der Mantelfläche (6) des Verankerungsteils (1) ein Aussengewinde (7) angebracht ist;
D) das Verankerungsteil (1) in Richtung der Längsachse (2) eine Höhe H aufweist und mit einer zur Längsachse (2) konzentrischen Bohrung (10), welche vom unteren Ende (4) her eine Tiefe T < H aufweist, versehen ist; und
E) das Verankerungsteil (1) am unteren Ende (4) selbstschneidend ausgebildet ist,
**dadurch gekennzeichnet, dass**
F) die Mantelfläche (6) vom unteren Ende (4) her auf einer Teillänge L < H eine glatte Oberfläche aufweist, und
G) die Teillänge L zwischen 50% und 85% der Höhe H beträgt.

2. Knochenverankerungselement (9) nach Anspruch 1, **dadurch gekennzeichnet, dass** am unteren Ende (4) des Verankerungsteiles (1) stirnseitig tangential angeordnete Schneidezähne (8) angebracht sind.

3. Knochenverankerungselement (9) nach Anspruch 2, **dadurch gekennzeichnet, dass** die Schnittkanten (11) der Schneidezähne (8) gegenüber einer durch einen Radius (25) und der zugehörigen Tangente (26) aufgespannten Ebene (31) einen Winkel (30) zwischen 30° und 60° einschliessen.

4. Knochenverankerungselement (9) nach einem der Ansprüche 1 bis 3, **dadurch gekennzeichnet, dass** die Schneidezähne (8) einen Spanwinkel von 10° bis 40° aufweisen.

5. Knochenverankerungselement (9) nach einem der Ansprüche 1 bis 3, **dadurch gekennzeichnet, dass** die Schneidezähne (8) einen Spanwinkel von 25° bis 35° aufweisen.

6. Knochenverankerungselement (9) nach einem der Ansprüche 1 bis 5, **dadurch gekennzeichnet, dass** die Schneidezähne (8) einen Freiwinkel von 15° bis 30° aufweisen.

7. Knochenverankerungselement (9) nach einem der Ansprüche 1 bis 6, **dadurch gekennzeichnet, dass** das Gewinde (7) selbstformend ist.

8. Knochenverankerungselement (9) nach einem der Ansprüche 1 bis 7, **dadurch gekennzeichnet, dass** die hohlzylindrische Seitenwand (12) des Verankerungsteiles (1) mit radialen Durchgangsöffnungen (13) versehen ist.

9. Vorrichtung zur Knochenfixation mit zwei Knochenverankerungselementen (9) nach einem der Ansprüche 1 bis 8, **dadurch gekennzeichnet, dass** sie
A) mindestens eine Platte (14;15) mit einer Zentralachse (16), wobei diese mindestens eine Platte (14:15) mit Mitteln (17) zur Aufnahme der Verbindungselemente (5) der beiden Knochenverankerungselemente (9) versehen ist; und
B) Mittel (18) zur Fixierung der Knochenverankerungselemente (9) in den Platten (14;15) umfasst, wobei
C) die beiden Knochenverankerungselemente (9) in Richtung der Zentralachse (16) verschiebbar sind.

10. Vorrichtung nach Anspruch 9, **dadurch gekennzeichnet, dass** die Vorrichtung zwei in Richtung der Zentralachse (16) verschiebbare Platten (14;15), wobei jede der beiden Platten (14:15) mit Mitteln (17) zur Aufnahme eines Verbindungselementes (5) versehen ist, und ein Feststellmittel (20) zur relativen Fixierung der beiden Platten (14;15) zueinander umfasst.

11. Vorrichtung nach Anspruch 9 oder 10, **dadurch gekennzeichnet, dass** die beiden Knochenverankerungselemente (9) entlang der Zentralachse (16) um einen Abstand Z gegeneinander verschiebbar sind.

12. Vorrichtung nach Anspruch 11, **dadurch gekennzeichnet, dass** der Abstand Z zwischen 20 und 60 mm beträgt.

13. Vorrichtung nach Anspruch 12, **dadurch gekennzeichnet, dass** der Abstand Z zwischen 30 und 55 mm beträgt.

14. Vorrichtung nach einem der Ansprüche 9 bis 13, **dadurch gekennzeichnet, dass** die beiden Platten (14;15) an den mit einander in Kontakt stehenden Flächen (21;22) mit einer Verzahnung (23) versehen sind.

15. Vorrichtung nach einem der Ansprüche 9 bis 14, **dadurch gekennzeichnet, dass** zwischen der Zentralachse (16) der mindestens einen Platte (14;15) und jeder der Längsachsen (2) der Verankerungsteile (1) ein Winkel zwischen 70° und 110° einstellbar ist.

## Claims

1. A bone anchorage element (9) including
A) a longitudinal, circularly cylindrical anchorage member (1) having a longitudinal axis (2), a top end portion (3), and a bottom end portion (4); as well as
B) a connecting member (5) arranged on the top end portion (3) for coupling with internal plates or longitudinal carriers;
C) the lateral area (6) of the anchorage member (1) being provided with an external screw thread (7);
D) the anchorage member (1) having a height H and being provided with a bore (10) with a depth T < H which begins at the bottom end portion (4) and extends concentrically to the longitudinal axis (2); and
E) the bottom end portion (4) of the anchorage member (1) being self-cutting,
**characterised in that**
F) the lateral area (6) is provided with a smooth surface beginning at the bottom end portion (4) and extending over a longitudinal portion L < H, and
G) the length of the longitudinal portion L is between 50 and 85 percent of the height H.

2. A bone anchorage element (9) as claimed in claim 1, **characterised in that** the bottom end portion (4) of the anchorage member (1) is provided with end-cutting, tangentially arranged cutting teeth.

3. A bone anchorage element (9) as claimed in claim 2, **characterised in that** the cutting edges (11) of the cutting teeth (8) form an angle (30) of between 30 and 60 degrees relative to a plane (31) defined by a radius (25) and the corresponding tangent (26).

4. A bone anchorage element (9) as claimed in any of the claims 1 to 3, **characterised in that** the cutting teeth (8) have an effective cutting angle of between 10 and 40 degrees.

5. A bone anchorage element (9) as claimed in any of the claims 1 to 3, **characterised in that** the cutting teeth (8) have an effective cutting angle of between 25 and 35 degrees.

6. A bone anchorage element (9) as claimed in any of the claims 1 to 5, **characterised in that** the cutting teeth (8) have a clearance angle of between 15 and 30 degrees.

7. A bone anchorage element (9) as claimed in any of the claims 1 to 6, **characterised in that** the thread (7) is self-tapping.

8. A bone anchorage element (9) as claimed in any of the claims 1 to 7, **characterised in that** the hollow, cylindrical lateral wall (12) of the anchorage member (1) is provided with radial through holes (13).

9. A bone fixation device including two bone anchorage elements (9) as claimed in any of the claims 1 to 8, **characterised in that** it comprises
A) at least one plate (14;15) having a central axis (16), said at least one plate (14;15) being provided with means (17) for receiving the connecting members (5) of the two bone anchorage elements (9);
B) means (18) for locking the bone anchorage elements (9) within the plates (14;15),
C) the two bone anchorage elements (9) being displaceable in the direction of the central axis (16).

10. A device as claimed in claim 9, **characterised in that** the device comprises two plates (14;15) displaceable in the direction of the central axis (16), each of the two plates (14;15) comprising means (17) for receiving a connecting member (5), and a locking means (20) for fixing the two plates (14;15) in their respective positions relative to each other.

11. A device as claimed in claim 9 or 10, **characterised in that** the two bone anchorage elements (9) are displaceable by a distance Z relative to each other along the central axis (16).

12. A device as claimed in claim 11, **characterised in that** the distance Z is between 20 and 60 mm.

13. A device as claimed in claim 12, **characterised in that** the distance Z is between 30 and 55 mm.

14. A device as claimed in any of the claims 9 to 13, **characterised in that** the two plates (14;15) on their surfaces (21;22) being in contact with each other are provided with a toothing (23).

15. A device as claimed in any of the claims 9 to 14, **characterised in that** between the central axis (16) of the at least one plate (14;15) and each of the longitudinal axes (2) of the anchorage members (1) an angle of between 70 and 110 degrees may be adjusted.

## Revendications

1. Elément d'ancrage dans l'os (9) comportant
A) une partie longitudinale d'ancrage (1) en forme de cylindre circulaire présentant un axe longitudinal (2), une extrémité supérieure (3) et une extrémité inférieure (4) ; ainsi que
B) un élément de liaison (5) installé à l'extrémité supérieure (3) pour l'accouplement à des plaques internes ou à des supports longitudinaux ;
C) un filet extérieur (7) étant ménagé sur la surface d'enveloppe (6) de la partie d'ancrage (1);
D) la partie d'ancrage (1) présentant dans la direction de l'axe longitudinal (2) une hauteur H et un alésage (10) qui est concentrique par rapport à l'axe longitudinal (2) et qui présente à partir de l'extrémité inférieure (4) une profondeur T < H ; et
E) la partie d'ancrage (1) est configurée de manière à être autotaraudeuse à l'extrémité inférieure (4);
**caractérisé en ce que**
F) la surface d'enveloppe (6) présente partant de l'extrémité inférieure (4) une surface lisse sur une longueur partielle L < H, et
G) la longueur partielle L est comprise entre 50 % et 85 % de la hauteur H.

2. Elément d'ancrage dans l'os (9) selon la revendication 1, **caractérisé en ce que** des dents de découpe (8) disposées tangentiellement sont installées frontalement à l'extrémité inférieure (4) de la partie d'ancrage (1).

3. Elément d'ancrage dans l'os (9) selon la revendication 2, **caractérisé en ce que** les bords de découpe (11) des dents de découpe (8) forment par rapport à un plan (31) sous-tendu par un rayon (25) et la tangente (26) associée un angle (30) compris entre 30° et 60°.

4. Elément d'ancrage dans l'os (9) selon l'une des revendications 1 à 3, **caractérisé en ce que** les dents de découpe (8) présentent un angle de découpe de 10° à 40°.

5. Elément d'ancrage dans l'os (9) selon l'une des revendications 1 à 3, **caractérisé en ce que** les dents de découpe (8) présentent un angle de découpe de 25° à 35°.

6. Elément d'ancrage dans l'os (9) selon l'une des revendications 1 à 5, **caractérisé en ce que** les dents de découpe (8) présente un angle libre de 15° à 30°.

7. Elément d'ancrage dans l'os (9) selon l'une des revendications 1 à 6, **caractérisé en ce que** le filet (7) est autoformant.

8. Elément d'ancrage dans l'os (9) selon l'une des revendications 1 à 7, **caractérisé en ce que** la paroi latérale (12) en forme de cylindre creux de la partie d'ancrage (1) est dotée d'ouverture radiale de passage (13).

9. Dispositif de fixation osseuse doté de deux éléments d'ancrage dans l'os (9) selon l'une des revendications 1 à 8, **caractérisé en ce qu'**il comprend
A) au moins une plaque (14 ; 15) dotée d'un axe central (16), cette plaque (14 ; 15) au moins présente étant dotée de moyens (17) de réception des éléments de liaison (5) de deux éléments d'ancrage dans l'os (9) ; et
B) des moyens (18) de fixation des éléments d'ancrage dans l'os (9) dans les plaques (14;15), et
C) les deux éléments d'ancrage dans l'os (9) peuvent coulisser dans la direction de l'axe central (16).

10. Dispositif selon la revendication 9, **caractérisé en ce que** le dispositif comprend deux plaques (14; 15) aptes à coulisser dans la direction de l'axe central (16), chacune des deux plaques (14; 15) étant dotées de moyens (17) de réception d'un élément de liaison (5), et un moyen d'immobilisation (20) pour la fixation mutuelle des deux plaques (14 ; 15).

11. Dispositif selon la revendication 9 ou 10, **caractérisé en ce que** les deux éléments d'ancrage dans l'os (9) peuvent coulisser l'un par rapport à l'autre sur une distance Z le long de l'axe central (16).

12. Dispositif selon la revendication 11, **caractérisé en ce que** la distance Z est comprise entre 20 et 60 mm.

13. Dispositif selon la revendication 12, **caractérisé en ce que** la distance Z est comprise entre 30 et 55 mm.

14. Dispositif selon l'une des revendications 9 à 13, **caractérisé en ce que** les deux plaques (14 ; 15) sont dotées d'une denture (23) sur leurs surfaces (21 ; 22) en contact l'une avec l'autre.

15. Dispositif selon l'une des revendications 9 à 14, **caractérisé en ce qu'**un angle compris entre 70° et 110° peut être ajusté entre l'axe central (16) de la plaque (14 ; 15) au moins présente et les axes longitudinaux (2) de chacune des parties d'ancrage (1).
